# EUROPEAN PATENT SPECIFICATION

(11) **EP 1 790 369 B1**
(45) Date of publication and mention of the grant of the patent: **03.06.2009**
(21) Application number: 06021981.3
(22) Date of filing: 19.10.2006
(51) Int. Cl.: A61M 16/06, A62B 17/04, A61M 16/20

(54) **Device for artificial respiration at multiple pressure levels**
Vorrichtung zur künstlichen Beatmung auf mehreren Druckstufen
Dispositif de respiration artificielle à des niveaux de pression multiples

(30) Priority: 24.10.2005 IT MI20052020
(43) Date of publication of application: 30.05.2007
(73) Proprietor: Starmed S.p.A., 41037 Mirandola MO (IT)
(72) Inventor: Pesenti, Antonio, 20133 Milano (IT); Patroniti, Nicolo, 20035 Lissone (IT)
(74) Representative: Alagem Modiano, Lara S.

(56) References cited:
- EP-A- 1 170 026
- WO-A-02/089886
- WO-A-02/096342
- WO-A-03/097145
- US-A- 4 773 411
- US-A1- 2005 098 179
- US-B1- 6 854 459

## Description

The present invention relates to a device for artificial respiration at multiple pressure levels.

As is known, artificial respiration therapies typically use so-called CPAP, ventilation with continuous positive pressure, obtained by means of a flowmeter which feeds a preset flow-rate of air at a preset pressure level.

In many cases it has been found that it is ideal to use two different levels of pressure during ventilation, and therefore solutions in which ventilation occurs alternately with two different pressure values have already been introduced.

US 6,854,459 B1 shows the features of the preamble of claim 1.

However, such solutions suffer several drawbacks, the first of which is that as the pressure level varies, so does the flow-rate of the air; moreover, in order to obtain the two different pressure levels it is necessary to use complex and expensive ventilation units.

The aim of the invention is to solve the problem described above by providing a device for artificial respiration at multiple pressure levels which allows to keep unchanged the flow-rate as the positive pressure level applied to the patient changes.

Within this aim, an object of the invention is to provide a device in which it is possible to use traditional flowmeters to provide the air stream, thus contributing to a general simplification of the apparatus, such as to allow use of the apparatus for home treatment.

Another object of the present invention is to provide a device which, thanks to its particular constructive characteristics, is capable of giving the greatest assurances of reliability and safety in use.

Still another object of the present invention is to provide a device which can be obtained easily starting from commonly commercially available elements and materials and is also competitive from a merely economical standpoint.

This aim and these and other objects, which will become better apparent hereinafter, are achieved by a device for artificial respiration at multiple pressure levels, which comprises a hood for accomodating the head of a patient, said hood being provided with at least one air intake connected to a ventilation unit and with an air outlet, characterized in that it comprises, operatively connected to said hood, at least two air discharge valves, which are set to two different pressure levels and can be activated selectively.

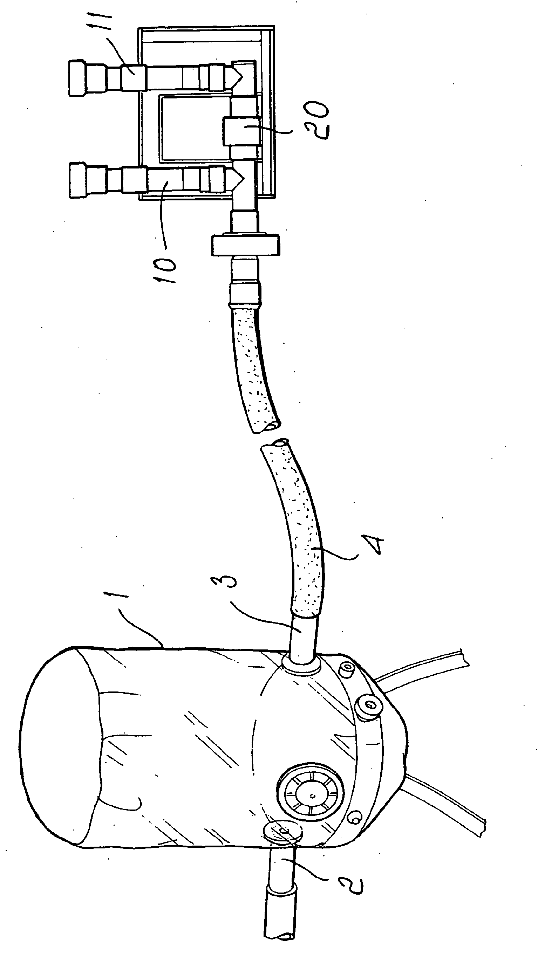

Further characteristics and advantages of the present invention will become better apparent from the description of a preferred but not exclusive embodiment of a device for artificial respiration at multiple levels, illustrated by way of non-limiting example in the accompanying drawing, wherein the only figure is a schematic view of the device according to the invention.

With reference to the only figure, the device for artificial respiration at multiple levels according to the invention comprises a hood 1 of a per se known type, which is applied substantially hermetically at the head of a patient.

The hood 1 is provided with at least one air intake 2, which is connected to a ventilation unit constituted advantageously by a traditional flowmeter or optionally by a Venturi meter.

An air outlet 3 is connected to the hood 1 and an air discharge duct 4 is connected to the outlet 3 and controlled by at least two discharge valves and preferably a first discharge valve 10 and a second discharge valve 11, which are set to two different pressure levels.

The valves 10 and 11, which theoretically can also be connected directly to the hood, are activated selectively so that it is possible to obtain the two pressure levels, in practice by varying the contrast pressure defined by the discharge valve.

In a preferred embodiment, the first valve 10, which is set to pressure level 10, is provided on a branch from the duct 4, and on the duct a closure valve 20 is provided, which is arranged upstream with respect to the second discharge valve 11, which is set to a lower pressure level.

With the described arrangement, therefore, by keeping open the closure valve 20, air tends to flow out from the second discharge valve, which is set to a lower level, whereas by closing the closure valve 20 the air is forced to exit from the first discharge valve 10, which is set to a higher pressure level; by acting appropriately on the closure valve 20 it is possible to alternate in succession the levels of pressure inside the hood, thus achieving a ventilation with continuous positive pressure at two different levels; the closure valve can act on the discharge valves 10 and 11 with closure times which can be differentiated in any manner.

This aspect is particularly important, since it allows to obtain the two levels particularly quickly and simply by using ordinary ventilation flowmeters.

If necessary, it is also optionally possible to provide a plurality of discharge valves, which can be activated selectively and are actuated according to different discharge pressures.

The invention thus conceived is susceptible of numerous modifications and variations, all of which are within the scope of the appended claims.

All the details may further be replaced with other technically equivalent elements.

In practice, the materials used, as well as the contingent shapes and dimensions, may be any according to requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for artificial respiration at multiple pressure levels, comprising a hood (1) for accomodating the head of a patient, said hood being provided with at least one air intake (2) connected to a ventilation unit and with an air outlet (3), **characterized in that** it comprises, operatively connected to said hood, at least two air discharge valves (10, 11), which are set to two different pressure levels and can be activated selectively.

2. The device according to claim 1, **characterized in that** it comprises an air discharge duct (4) which can be associated with said air outlet (3), said at least two air discharge valves (10, 11) being connected to said duct.

3. The device according to one or more of the preceding claims, **characterized in that** it comprises a first valve (10) and a second valve (11), which are arranged as a branch on said air discharge duct and are arranged in succession along the direction of the outflowing air.

4. The device according to one or more of the preceding claims, **characterized in that** said first air discharge valve (10) is set to a higher pressure level than said second discharge valve (11).

5. The device according to one or more of the preceding claims, **characterized in that** it comprises, on said discharge duct (4), between said first and second valves (10, 11), a controlled closure valve (20) for opening and closure.

6. The device according to one or more of the preceding claims, **characterized in that** said ventilation unit is constituted by a ventilation flowmeter.

7. The device according to one or more of the preceding claims, **characterized in that** said ventilation unit is constituted by a Venturi meter.

## Patentansprüche

1. Eine Vorrichtung zur künstlichen Beatmung bei eine Vielzahl von Druckstufen, die eine Haube (1) zur Aufnahme des Kopfs eines Patienten umfasst, wobei die Haube mit mindestens einem Lufteinlass (2) versehen ist, der mit einer Belüftungseinheit verbunden ist, und mit einem Luftauslass (3) versehen ist, **dadurch gekennzeichnet, dass** sie, operativ mit der Haube verbunden, mindestens zwei Luftauslassventile (10, 11) umfasst, die auf zwei verschiedene Druckstufen eingestellt sind und selektiv aktiviert werden können.

2. Die Vorrichtung gemäß Anspruch 1, **dadurch gekennzeichnet, dass** sie einen Luftablasskanal (4) umfasst, der mit dem Luftauslass (3) verbunden werden kann, wobei die mindestens zwei Luftauslassventile (10, 11) mit dem Kanal verbunden sind.

3. Die Vorrichtung gemäß einem oder mehreren der obigen Ansprüche, **dadurch gekennzeichnet, dass** sie ein erstes Ventil (10) und ein zweites Ventil (11) umfasst, die als Verzweigung an dem Luftablasskanal angeordnet und nacheinander entlang der Richtung der herausströmenden Luft angeordnet sind.

4. Die Vorrichtung gemäß einem oder mehreren der obigen Ansprüche, **dadurch gekennzeichnet, dass** das erste Luftauslassventil (10) auf eine höhere Druckstufe eingestellt ist als das zweite Auslassventil (11).

5. Die Vorrichtung gemäß einem oder mehreren der obigen Ansprüche, **dadurch gekennzeichnet, dass** sie, an dem Ablasskanal (4), zwischen dem ersten und dem zweiten Ventil (10, 11), ein gesteuertes Verschlussventil (20) zum Öffnen und zum Schließen umfasst.

6. Die Vorrichtung gemäß einem oder mehreren der obigen Ansprüche, **dadurch gekennzeichnet, dass** die Belüftungseinheit aus einem Belüftungs-Durchflussmesser besteht.

7. Die Vorrichtung gemäß einem oder mehreren der obigen Ansprüche, **dadurch gekennzeichnet, dass** die Belüftungseinheit aus einer Venturi-Messdüse besteht.

## Revendications

1. Dispositif de respiration artificielle à niveaux de pression multiples, comprenant un capot (1) pour recevoir la tête d'un patient, ledit capot étant muni d'au moins une entrée d'air (2) raccordée à une unité de ventilation et une sortie d'air (3), **caractérisé en ce qu'**il comprend, reliées de manière opérationnelle audit capot, au moins deux soupapes d'évacuation d'air (10, 11), qui sont réglées à deux niveaux de pression différents et qui peuvent être activées sélectivement.

2. Dispositif selon la revendication 1, **caractérisé en ce qu'**il comprend une conduite d'évacuation d'air (4) qui peut être associée à ladite sortie d'air (3), lesdites au moins deux soupapes d'évacuation d'air (10, 11) étant raccordées à ladite conduite.

3. Dispositif selon une ou plusieurs des revendications précédentes, **caractérisé en ce qu'**il comprend une première soupape (10) et une seconde soupape (11), qui sont disposées sous la forme d'une dérivation sur ladite conduite d'évacuation d'air et qui sont disposées à la suite le long de la direction de l'air s'écoulant vers la sortie.

4. Dispositif selon une ou plusieurs des revendications précédentes, **caractérisé en ce que** ladite première soupape d'évacuation d'air (10) est réglée à un niveau de pression plus élevé que ladite seconde soupape d'évacuation (11).

5. Dispositif selon une ou plusieurs des revendications précédentes, **caractérisé en ce qu'**il comprend, sur ladite conduite d'évacuation (4), entre lesdites première et seconde soupapes (10, 11), une soupape à fermeture commandée (20) pour l'ouverture et la fermeture.

6. Dispositif selon une ou plusieurs des revendications précédentes, **caractérisé en ce que** ladite unité de ventilation est constituée d'un débitmètre de ventilation.

7. Dispositif selon une ou plusieurs des revendications précédentes, **caractérisé en ce que** ladite unité de ventilation est constituée d'un compteur venturi.
